# DEMANDE DE BREVET EUROPEEN

(11) **EP 1 369 166 A1**
(43) Date de publication de la demande: **10.12.2003**
(21) Numéro de dépôt: 03291117.4
(22) Date de dépôt: 14.05.2003
(51) Int. Cl.: B01D 71/02, B01D 53/22, B01D 69/10, B01J 35/06, B01J 37/02, C07C 7/144, C07C 7/13

(54) **Membrane zéolithique de faible epaisseur, sa préparation et son utilisation en séparation**

(30) Priorité: 03.06.2002 FR 0206817
(71) Demandeur: Institut Français du Pétrole, 92852 Rueil-Malmaison Cedex (FR)
(72) Inventeur: Chau, Christophe, 92500 Rueil Malmaison (FR); Sicard, Mickael, 91120 Palaiseau (FR); Le Dred, Ronan, 68400 Riedisheim (FR)

(57) **Abrégé**

On décrit une membrane zéolithique supportée constituée d'une couche composite zéolithe/support présentant, dans la séparation n-butane/isobutane, une perméance de n-butane d'au moins 6.10⁻⁷ mol/m².s.Pa et une sélectivité d'au moins 250 à la température de 180°C. Ladite couche composite zéolithe/support est fine et continue. Cette membrane est utilisée dans des procédés de séparation de gaz, de séparation de vapeurs ou de séparation de liquides.

## Description

La présente invention se rapporte au domaine des membranes zéolithiques supportées utilisées en séparation.

Elle a plus particulièrement pour objet une membrane zéolithique supportée, un procédé pour sa préparation et son utilisation en séparation.

Divers procédés d'élaboration de membranes zéolithiques ont déjà été décrits. Il apparaît à ce jour difficile d'obtenir de manière contrôlée et reproductible des membranes zéolithiques dont la couche contenant la zéolithe est continue et fine. La finesse et la continuité d'une telle couche sont des paramètres essentiels pour obtenir un matériau membranaire présentant des propriétés intéressantes qui puissent être mises à profit dans des procédés de séparation industriels. Notamment, il est particulièrement difficile de maîtriser la préparation des membranes zéolithiques: les procédés d'obtention font intervenir plusieurs étapes et il est souvent nécessaire de reproduire l'étape de cristallisation à plusieurs reprises pour obtenir, à la suite d'étapes consommatrices en temps, en coûts opératoires, en produits chimiques et en énergie, une couche continue qui puisse être mise en oeuvre en séparation. Par ailleurs, la stabilité thermique et mécanique de ces membranes inorganiques est cruciale. En effet, les matériaux inorganiques, peuvent en général, être mis en oeuvre à des températures relativement élevées, par exemple plus élevées que les membranes polymères organiques qui opèrent généralement à une température inférieure à 100°C. Il est alors essentiel, pour une application industrielle et commerciale, de disposer d'une membrane qui puisse demeurer stable lors d'opérations et de mises en oeuvre à des températures élevées voire de pressions élevées. La voie hydrothermale mettant en jeu des supports poreux présente l'avantage de stabiliser les cristaux de zéolithes dans la porosité d'une matrice poreuse (alumine, inox par exemple) et à la surface de celle-ci.

Dans la demande de brevet EP-A-0 778 075, il est décrit un procédé d'élaboration de membranes de zéolithe supportée par du verre poreux. Le brevet US-A-5,429,743 et la demande de brevet internationale WO-A-95/29751 décrivent des protocoles d'obtention de membranes composites supportées par une matrice macroporeuse inorganique. On pourra également se référer aux documents US-A-4,099,692, WO-A-93/19840, US-A-5,567,664 et WO-A-96/01683. Dans la demande de brevet internationale WO-A-00/33948, il est décrit un procédé d'obtention de membranes composites de zéolithe supportée sur des solides tubulaires éventuellement multi-canaux. L'ensemble de ces matériaux membranaires composites à base de zéolithe est formé d'une phase zéolithique déposée sur un support. Une série de brevets récents (US 5,871,650, US 5,968,366, US 6,090,289, US 6,074,457, WO-A-00/53297, WO-A-00/53298) décrit la préparation de membranes dont la phase zéolithique MFI se trouve à la surface externe d'un support poreux. La cristallisation de la zéolithe s'effectue généralement par des traitements hydrothermaux multiples d'un mélange contenant les précurseurs de la phase zéolithique, ce qui augmente l'épaisseur effective de la couche séparatrice. Lorsque l'étape de cristallisation de la zéolithe est reproduite à plusieurs reprises, la synthèse est reproduite après retour éventuel du matériau à température ambiante, lavage et séchage dudit matériau.

La répétition et la succession d'opérations identiques pour la préparation de membranes zéolithiques permettent le dépôt de couches successives et/ou la formation de cristaux de zéolithes qui comblent les espaces interparticulaires, ce qui permet l'obtention d'une couche continue pour la séparation. Ce mode de synthèse en plusieurs étapes, s'il conduit à l'obtention d'une couche continue, conduit également à l'obtention de couches épaisses de zéolithes, qui risquent de se fissurer lors de la calcination de la membrane (Vroon, Z.A.E.P., Keizer, K., Burggraaf, A.J., Verweij, H., J. Membr. Sci. 144 (1998) 65-76), de la mise en régime de l'unité de séparation par membrane ou de la mise en oeuvre à haute température. Par ailleurs, l'augmentation d'épaisseur peut considérablement limiter le transfert de matière à travers la membrane lors de l'opération de séparation et diminuer ainsi l'intérêt technique et économique de l'opération de séparation par membrane, dû à une réduction de productivité de ladite étape de séparation. De plus, une membrane dont la couche séparatrice est épaisse nécessitera de mettre en oeuvre des surfaces importantes dudit matériau membranaire pour traiter un débit de charge de mélange à séparer, ce qui se traduit par des investissements élevés. En outre, ce mode de synthèse en plusieurs étapes nécessite une quantité importante des précurseurs de la phase zéolithique, ce qui augmente notamment le coût des matières premières et précurseurs utilisés. Il présente également l'inconvénient d'allonger la durée d'obtention du matériau membranaire et d'augmenter le coût opératoire de la séparation.

Une des difficultés liées à la préparation de membranes à base de zéolithe réside notamment dans le contrôle de la cristallisation de la zéolithe afin d'obtenir des cristaux de zéolithe bien liés au support, localisés principalement dans la porosité du support, formant ainsi une couche composite zéolithe/support continue (obtenue en obstruant les espaces vides du support par des cristaux de phase zéolithique) et fine de manière à limiter la résistance de transfert à travers le matériau membranaire. La localisation majoritaire, de préférence intégrale, de la phase zéolithique dans la porosité du support confère une très bonne résistance thermique et mécanique au matériau membranaire. Toutefois, il n'est pas exclu qu'une partie minoritaire de la phase zéolithique soit localisée à la surface externe du support.

C'est un des objets essentiels de la présente invention que de fournir une membrane zéolithique supportée dans laquelle la phase zéolithique, cristallisée par un unique traitement hydrothermal, présente les caractéristiques énoncées ci-dessus. En particulier, ladite phase zéolithique, qui est active en séparation, c'est-à-dire sélective vis-à-vis des composés à séparer, est fine et présente en outre une cristallinité très élevée.

La membrane zéolithique supportée selon l'invention est constituée d'une couche composite zéolithe/support et se caractérise en ce qu'elle présente, dans la séparation n-butane/isobutane, une perméance de n-butane d'au moins 6. 10⁻⁷ mol/m².s.Pa et une sélectivité d'au moins 250 à la température de 180°C.

On rappelle, que, par définition, la perméance d'un gaz, exprimée en mol/m².s.Pa, est le débit molaire (mol/s) de ce gaz ramené à l'unité de surface (m²) membranaire et ramené à la différence de pression partielle de ce gaz entre l'amont (où circule la charge) et l'aval (où l'on récupère le perméat). La perméance d'un gaz est donc le débit molaire de ce gaz traversant la membrane par unité de surface et de pression. La sélectivité α (appelée permsélectivité) est, dans le cas de mesures de perméation de corps purs, le rapport des perméances de ces corps purs. Dans le cadre de la présente invention, la sélectivité est donc le rapport des perméances du n-butane et de l'isobutane.

De préférence, la membrane zéolithique supportée selon l'invention présente, dans la séparation n-butane/isobutane, une perméance de n-butane d'au moins 8.10⁻⁷ mol/m².s.Pa et de manière très préférée d'au moins 10.10⁻⁷ mol/m².s.Pa à la température de 180°C. La perméance élevée de n-butane des membranes selon l'invention témoignent la faible épaisseur de la phase zéolithique ainsi que celle de la couche composite zéolithe/support laquelle présente une épaisseur inférieure à 2 µm et de préférence inférieure à 1 µm et de manière très préférée inférieure à 0,5 µm.

La perméance est mesurée comme suit : la membrane est insérée dans un perméateur (module de mesure de perméation) grâce à des joints de carbone, qui maintiennent l'étanchéité de ce module de mesure. L'ensemble (module/membrane) est placé dans une unité de perméation gazeuse et le matériau est au préalable traité à 350 °C sous un débit de gaz inerte comme l'hélium ce qui permet d'éliminer toute trace de gaz adsorbable sur la surface externe et dans la porosité interne du matériau membranaire. Durant les mesures de perméation de gaz, la membrane est soumise à une différence de pression, la pression du côté amont où circule la charge (du butane linéaire n-C₄H₁₀ pur ou de l'isobutane pur i-C₄H₁₀) est maintenue constante à 1,5 bar (0,15 MPa) absolus et la pression du côté aval, où l'on récupère le perméat après extraction sélective d'une partie des molécules présentes dans la charge, est la pression atmosphérique. Cette différence de pression constitue la force motrice du transfert à travers la membrane. Le débit de gaz traversant la membrane est mesuré au moyen d'un débitmètre volumique. Le seuil de détection est inférieur à 0,002 mL/mn soit environ 10⁻⁶ mol/m².s de n-butane ou isobutane. La mesure des débits de gaz traversant la membrane est effectuée avec les corps purs n-butane et isobutane. Durant les mesures de perméation de gaz, la température est maintenue à 180°C.

Il est à noter qu'avec le choix de ces molécules sondes que sont le n-butane et l'isobutane, cette méthode de caractérisation est considérée comme un critère très sévère et très sélectif pour caractériser des membranes inorganiques microporeuses et en particulier des membranes zéolithiques. Elle permet dès lors de mettre en évidence la présence de toute discontinuité, de défauts, de fissures dans la couche composite zéolithe/support. A l'inverse, l'absence de défauts notables dans la membrane révèle un potentiel très élevé en séparation. En particulier, cette méthode de caractérisation utilisant le n-butane et l'isobutane est très sévère par rapport à d'autres tests de caractérisation employés dans l'art antérieur, par exemple les tests utilisant les couples N₂/SF₆, H₂/n-C₄ ou H₂/i-C₄.

De préférence, la membrane zéolithique supportée selon l'invention présente une sélectivité d'au moins 1000.

La membrane zéolithique supportée selon l'invention est constituée d'une couche composite zéolithe/support continue et fine dont la phase zéolithique est également fine, ce qui se traduit par des performances séparatrices très élevées, en particulier par une sélectivité ou pouvoir séparateur de la membrane très élevée, c'est-à-dire d'au moins 250, de préférence d'au moins 1000, voire infinie. Ladite couche composite zéolithe/support est active en séparation, c'est-à-dire sélective vis-à-vis des composés à séparer. Par ailleurs les membranes selon l'invention sont des matériaux composites, dont la couche sélective ou séparatrice est formée de cristaux de zéolithe immobilisés et stabilisés dans la porosité d'un support inorganique. La phase zéolithique est majoritairement, de préférence intégralement, localisée dans la porosité du support, ce qui confère une très bonne résistance thermique et mécanique au matériau membranaire. Cette phase zéolithique présente une cristallinité très élevée, de préférence d'au moins 85% et de manière très préférée d'au moins 90%. Les membranes zéolithiques selon l'invention présentent également une très bonne intégrité structurale, c'est-à-dire une absence de défauts dans la structure de la couche composite zéolithe/support et une absence d'espaces interparticulaires, c'est-à-dire de vides présents entre les cristaux de la zéolithe, ce qu'il est difficile d'obtenir par les procédés antérieurs en une seule étape. La finesse de la couche zéolithique n'empêche pas d'utiliser avantageusement la membrane selon l'invention à des températures élevées, notamment supérieures à 100°C (comme par exemple 180°C).

La phase zéolithique contenue dans ladite couche composite est de préférence de type structural MFI (silicalite-1 ou ZSM-5) avec des dimensions de canaux de 0.51*0.55 et 0.53*0.56 nm².

Le support compris dans la couche composite zéolithe/support de la membrane selon l'invention est constitué d'un matériau inorganique poreux. Un support en céramique à base d'alumine et/ou de zircone et/ou d'oxyde de titane est un support approprié. D'autres matériaux, dont la nature suit, peuvent également convenir: carbone, silice, zéolithes, argiles, verré poreux, métal poreux. L'utilisation d'un support en alumine de variété allotropique alpha ou gamma est préférée. Ce support peut se présenter, par exemple, sous forme plane ou tubulaire ou sous la forme de fibres creuses ou bien encore de monolithes multi-canaux. D'autres géométries peuvent convenir, mais les géométries de support compatibles avec une utilisation industrielle de ces membranes sont avantageusement employées. En particulier les supports tubulaires et les supports sous forme de fibres creuses permettent d'opérer des modules et des unités compactes (rapport surface de membrane/volume de l'équipement élevé) pour traiter des débits de charge importants.

La présente invention concerne également un procédé d'élaboration contrôlée des membranes zéolithiques selon l'invention.

Les membranes zéolithiques supportées selon l'invention sont avantageusement préparées par un procédé qui comprend :
a) la formation d'un gel ou d'une solution comprenant au moins une source de silice et de l'eau, additionné d'au moins un composé organique polaire;
b) la mise en contact dudit gel ou de ladite solution avec un support poreux ;
c) la cristallisation de la zéolithe à partir dudit gel ou de ladite solution ; et
d) l'élimination d'agents résiduels.

La source de silice employée dans l'étape (a) du procédé est de préférence une silice colloïdale ou une silice précipitée. Il peut également s'agir d'ions silicates tels que le silicate de sodium, d'alcoxydes de silicium ou du tétrachlorure de silicium.

D'autres éléments peuvent également être introduits en quantité minoritaire lors de l'étape (a) du procédé selon l'invention. En particulier, l'aluminium, le bore, le gallium, le titane, le germanium et le phosphore ainsi que le mélange de ces éléments peuvent être ajoutés lors de l'étape (a).

Le composé organique polaire additionné au gel ou à la solution comprenant au moins ladite source de silice et l'eau est de préférence un composé basique. Il s'agit avantageusement d'hydroxydes organiques, tel que l'hydroxyde de tétrapropylammonium, d'agents structurants organiques contenant des paires ioniques (ions ammonium ou phosphonium et les anions correspondants) ou des molécules neutres (aminés, alcools ou éthers tels que les éthers-couronnes et les cryptands). Le rapport molaire du composé organique polaire à la silice est compris entre 0,3:1 et 0,6:1 et de préférence entre 0,35:1 et 0,50:1. Les ions hydroxydes ou fluorures peuvent par ailleurs être utilisés pour la dissolution des précurseurs et sont introduits dans le milieu de préparation par exemple sous forme d'hydroxyde de sodium, d'hydroxydes organiques et d'acide fluorhydrique.

La dilution de la source de silice dans la solution ou le gel utilisé dans l'étape (a) et la durée de cristallisation de la zéolithe dans l'étape (c) sont des paramètres essentiels à maîtriser pour atteindre les propriétés désirées de la phase zéolithique déposée dans la porosité du support.

Un mode de réalisation particulier du procédé selon l'invention consiste à utiliser une silice précipitée comme source de silice dans l'étape (a), dans un rapport molaire de l'eau à la silice compris entre 45:1 et 65:1 et à appliquer dans l'étape (c) une durée de cristallisation inférieure ou égale à 80 heures.

Un autre mode de réalisation particulier du procédé selon l'invention consiste à utiliser une silice colloïdale comme source de silice dans l'étape (a), dans un rapport molaire de l'eau à la silice compris entre 18:1 et 35:1 et à appliquer dans l'étape (c) une durée de cristallisation inférieure ou égale à 45 heures.

Conformément à l'invention, la cristallisation de la zéolithe dans l'étape (c) du procédé selon l'invention est réalisée en une seule étape, c'est-à-dire que la zéolithe est cristallisée par un unique traitement hydrothermal.

L'élimination des agents résiduels, principalement du composé organique polaire, conformément à l'étape (d) du procédé selon l'invention, est effectuée par traitement thermique réalisé entre 350 et 550°C, de préférence entre 400°C et 500°C, dans un four sous atmosphère d'air ou sous atmosphère N₂/O₂ dans des proportions variables. Après élimination de ces agents résiduels, la microporosité des membranes zéolithiques peut alors être mise en oeuvre pour une opération de séparation. La calcination à haute température, c'est-à-dire réalisée entre 350 et 550°C, de préférence entre 400°C et 500°C, de la membrane afin d'éliminer les agents résiduels n'a aucune influence sur les performances séparatrices de la membrane, lesquelles demeurent très satisfaisantes. Cette très bonne résistance thermique de la membrane est avantageusement exploitée pour réaliser des séparations à hautes températures, dans des domaines où les membranes organiques ne peuvent être opérées du fait de leur faible résistance thermique. Les membranes zéolithiques selon l'invention apparaissent donc particulièrement adaptées pour des séparations à hautes comme à basses températures, dans des procédés industriels qui nécessitent de traiter des quantités de charge parfois importantes, tout en limitant l'investissement requis (membranes de hautes perméabilités).

Aussi, les membranes zéolithiques selon l'invention peuvent être utilisées pour différentes séparations moléculaires. Elles sont avantageusement utilisées dans des procédés de séparation de gaz, de séparation de vapeurs ou de séparation de liquides. Ainsi, elles sont préférentiellement utilisées pour séparer :
- des paraffines linéaires et branchées (n- et iso-paraffines), comme par exemple le n-butane et l'isobutane,
- des paraffines branchées entre elles (mono-branchées et di-branchées ou multi-branchées),
- des oléfines linéaires et branchées (n- et iso-oléfines),
- des paraffines et des oléfines,
- des naphtènes et des paraffines,
- des paraffines et des aromatiques,
- de l'hydrogène et des hydrocarbures ; par exemple, dans les mélanges contenant l'hydrogène et les hydrocarbures suivants, présents séparément ou simultanément, les paraffines légères comme le méthane, l'éthane, le propane ou le butane et l'isobutane ; ou encore l'hydrogène et les oléfines légères comme l'éthylène, le propylène et les isomères des butènes, l'isobutène ; ou bien encore l'hydrogène et les hydrocarbures polyinsaturés comme l'acétylène, le propyne, butyne et butadiène, ces hydrocarbures étant pris séparément avec l'hydrogène ou en mélange,
- les isomères du xylène (ortho-, méta-, para-xylènes)
- le méthane et le CO₂.

Conformément à l'invention, la qualité et les propriétés de tamisage moléculaire de la phase zéolithique sont mises à profit pour séparer des molécules dont les dimensions sont inférieures strictement d'une part et d'autre part supérieures strictement à celles des pores de la zéolithe (séparation par différentiation de taille). A titre illustratif, dans le cas de la zéolithe MFI qui présente une taille moyenne de pores de 0,55 nm (dimensions de canaux de 0.51*0.55 et 0.53*0.56 nm²), les membranes zéolithiques selon l'invention peuvent être utilisées pour la séparation de molécules, en particulier contenant des atomes de carbone et d'hydrogène, dont les dimensions sont d'une part inférieures à 0,45 nm environ et d'autre part supérieures à 0,55 nm. Par ailleurs, les interactions entre les molécules à séparer et la phase zéolithique de la membrane peuvent également être mises à profit pour réaliser la séparation desdites molécules (séparation par adsorption).

Les exemples suivants illustrent l'invention et ne doivent en aucune manière être considérés comme limitatifs.

### Exemple 1 : préparation d'une membrane zéolithique (conforme à l'invention)

On prépare une solution précurseur de zéolithe, à partir d'une source de silice (Aérosil 380® de surface spécifique égale à 380 m²/g commercialisé par la société Degussa), mélangée à température ambiante à de l'eau distillée et une solution aqueuse molaire de l'agent organique polaire d'hydroxyde d'ammonium tétrapropylé TPAOH (Fluka). La solution résultante admet pour composition molaire : 1 SiO₂: 0,4 TPAOH : 63,7 H₂O. Cette solution est laissée mûrir sous vive agitation durant 72 heures, ce qui permet une dépolymérisation partielle et une réorganisation de la silice en espèces silicates plus réactives que la source d'origine. Une gélification partielle des précurseurs peut intervenir, la solution étant plus ou moins limpide. Un support poreux d'alumine alpha (commercialisée par la société Exekia), préalablement lavé à l'eau distillée puis séché à 60°C, est ensuite immergé dans la solution préparée précédemment. L'ensemble des précurseurs et le support poreux sont placés dans un autoclave étanche, inséré dans une étuve maintenue à 175°C durant 60 heures. Après retour à température ambiante, la membrane est recueillie et lavée à l'eau distillée puis séchée à 60°C. L'élimination des agents résiduels, principalement l'hydroxyde d'ammonium tétrapropylé TPAOH, est effectuée par traitement thermique à 480°C dans un four sous atmosphère d'air. Après élimination de ces agents résiduels, la microporosité des membranes zéolithiques peut alors être mise en oeuvre pour une opération de séparation. L'analyse de phase par diffraction des rayons X sur cette membrane obtenue après 60 heures de séjour en autoclave confirme la présence de cristaux de zéolithe MFI localisés dans la porosité du support d'alumine alpha.

### Exemple 2: préparation d'une membrane zéolithique (conforme à l'invention)

Le protocole de préparation est analogue à celui décrit dans l'exemple 1 mais la préparation est menée en présence d'une source de silice colloïdale, le Bindzil 40/130® (commercialisée par la société Akzo Nobel). On procède ainsi à la préparation d'une membrane zéolithique pour laquelle la solution précurseur de la zéolithe MFI, admet respectivement pour stoechiométrie molaire 1 SiO₂ : 0,4 TPAOH : 18,3 H₂O. La durée de cristallisation est de 30 heures. L'élimination des agents résiduels, principalement l'hydroxyde d'ammonium tétrapropylé TPAOH, est effectuée par traitement thermique à 480°C dans un four sous atmosphère d'air. Après élimination de ces agents résiduels, la microporosité des membranes zéolithiques peut alors être mise en oeuvre pour une opération de séparation. L'analyse de phase par diffraction des rayons X sur cette membrane obtenue après 30 heures de séjour en autoclave confirme la présence de cristaux de zéolithe MFI localisés dans la porosité du support d'alumine alpha.

### Exemple 3 : préparation de membranes zéolithiques (non conformes à l'invention)

### a) utilisation d'une source de silice précipitée:

Le protocole opératoire décrit dans l'exemple 1 est repris en modifiant uniquement la quantité d'eau introduite dans la solution précurseur de la zéolithe MFI et la durée de cristallisation. La source de silice est l'Aérosil 380®. La durée de cristallisation est de 72 heures et l'élimination de l'hydroxyde d'ammonium tétrapropylé TPAOH est effectuée à 480°C.

On procède ainsi à la préparation d'une membrane zéolithique pour laquelle la solution précurseur de la zéolithe MFI, admet respectivement pour stoechiométrie molaire 1 SiO₂: 0,4 TPAOH : 29,6 H₂O. L'analyse de phase par diffraction des rayons X sur cette membrane obtenue après 72 heure de séjour en autoclave confirme la présence de cristaux de zéolithe MFI localisés dans la porosité du support d'alumine alpha.

### b) utilisation d'une source de silice colloïdale:

Le protocole opératoire décrit dans l'exemple 2 est repris en modifiant uniquement la durée de cristallisation. La source de silice est le Bindzil 40/130®. La durée de cristallisation est de 72 heures et l'élimination de l'hydroxyde d'ammonium tétrapropylé TPAOH est effectuée à 480°C.

On procède ainsi à la préparation d'une membrane zéolithique pour laquelle la solution, précurseur de la zéolithe MFI, admet respectivement pour stoechiométrie molaire i SiO₂: 0,4 TPAOH : 18,3 H₂O. L'analyse de phase par diffraction des rayons X sur cette membrane obtenue après 72 heure de séjour en autoclave confirme la présence de cristaux de zéolithe MFI localisés dans la porosité du support d'alumine alpha.

### Exemple 4 : performances séparatrices des matériaux membranaires zéolithiques (conformes à l'invention)

Cinq membranes (A, B, C, D, E) sont préparées selon le mode de synthèse décrit dans l'exemple 1. La membrane E est obtenue selon le mode de synthèse décrit dans l'exemple 1 mais avec une durée de cristallisation de 72 heures.

On teste également les performances d'une membrane F préparée selon le mode de synthèse décrit dans l'exemple 2.

Les performances des membranes A, B, C, D, E et F selon l'invention sont mises en évidence par mesure de séparation de gaz (perméation gazeuse), réalisée en utilisant le n-butane et l'isobutane selon le protocole décrit plus haut dans la description.

Les résultats sont rassemblés dans le tableau 1.

**Tableau 1 :**

| caractérisation des performances des membranes zéolithiques selon l'invention à 180°C | | | |
|---|---|---|---|
| Membrane | Perméance nC₄H₁₀ (10⁻⁷ mol/m².s.Pa) | Perméance iC₄H₁₀ (10⁻⁷ mol/m².s.Pa) | **Sélectivité nC**_{**4**}**H**_{**10**}**C**_{**4**}**H**_{**10**} |
| A | 11,62 | 0,000 | **Infinie** |
| B | 16,91 | 0,042 | **400** |
| C | 17,16 | 0,061 | **282** |
| D | 20,95 | 0,000 | **Infinie** |
| E | 7,10 | 0,000 | **Infinie** |
| F | 9,66 | 0,000 | **Infinie** |

Les membranes zéolithiques selon l'invention présentent, à l'issue des mesures de caractérisation, des perméances très élevées, la plupart supérieures à 10.10⁻⁷mol/m².s.Pa de n-butane à la température de 180°C, ce qui traduit une épaisseur très faible des membranes zéolithiques A, B, C, D, E et F. Dans les mêmes conditions, ces matériaux sont imperméables à l'isobutane (A, D, E, F) ou leurs perméances sont très faibles (B et C), dans tous les cas inférieures à 0,065.10⁻⁷ mol/m².s.Pa d'isobutane. Par suite, la sélectivité n-butane / isobutane ou nC₄H₁₀/iC₄H₁₀ atteint des valeurs particulièrement élevées, dans tous les cas supérieures à 250 et la séparation est particulièrement efficace, ce qui témoigne de la continuité de la couche composite zéolithe / support de chacune des membranes selon l'invention. Cette couche est sélective pour séparer des isomères d'hydrocarbures dont les dimensions ne diffèrent que de 0,6 nanomètre (diamètre cinétique de 0,43 nm pour le n-butane et de 0,49 nm pour l'isobutane). Par comparaison, il est généralement admis dans la littérature que des membranes zéolithiques de type structural MFI présentent une bonne intégrité texturale, c'est-à-dire une absence de défauts de structure de type mésopore et macropore, lorsque la sélectivité n-butane/isobutane est supérieure à 10 (Vroon et al., J. Membr. Sci. 113 (1996) 293).

### Exemple 5 : performances séparatrices de matériaux membranaires zéolithiques (non conformes à l'invention)

La membrane préparée selon l'exemple 3a (membrane G) et celle préparée selon l'exemple 3b (membrane H) sont soumises au même test de performances (perméation gazeuse) que les membranes selon l'invention préparées selon les exemples 1 et 2. Les conditions de réalisation de la perméation gazeuse avec le n-C₄ et iC₄ sont analogues à celles explicitées plus haut dans la description.
Les résultats sont rassemblés dans le tableau 2.

**Tableau 2 :**

| caractérisation des performances des membranes zéolithiques G et H à 180°C | | | |
|---|---|---|---|
| Membrane | Perméance nC₄H₁₀ (10⁻⁷mol/m².s.Pa) | Perméance iC₄H₁₀ (10⁻⁷mol/m².s.Pa) | **Sélectivité nC**_{**4**}**H**_{**10**}**/iC**_{**4**}**H**_{**10**} |
| G | 3,18 | 0,000 | **Infinie** |
| H | 1,84 | 0,000 | **Infinie** |

Les membranes G et H présentent dans la séparation n-C4/i-C4 des perméances de n-butane bien inférieures à celles des membranes selon l'invention. Les membranes selon l'invention présentent en effet une perméance de n-butane environ 2 à 11 fois plus élevée que celle des membranes non conformes à l'invention, tout en conservant des sélectivités élevées voire infinie. En d'autres termes, les membranes selon l'invention contiennent une couche composite zéolithe/support pouvant être jusqu'à au moins 11 fois plus fine que celle des membranes non conformes.

## Revendications

1. Membrane zéolithique supportée constituée d'une couche composite zéolithe/support **caractérisée en ce qu'**elle présente, dans la séparation n-butane/isobutane, une perméance de n-butane d'au moins 6.10⁻⁷ mol/m².s.Pa et une sélectivité d'au moins 250 à la température de 180°C.

2. Membrane zéolithique supportée selon la revendication 1 **caractérisée en ce qu'**elle présente, dans la séparation n-butane/isobutane, une perméance de n-butane d'au moins 8.10⁻⁷ mol/m².s.Pa à la température de 180°C.

3. Membrane zéolithique supportée selon la revendication 2 **caractérisée en ce qu'**elle présente, dans la séparation n-butane/isobutane, une perméance de n-butane d'au moins 10.10⁻⁷ mol/m².s.Pa à la température de 180°C.

4. Membrane zéolithique supportée selon l'une des revendications 1 à 3 **caractérisée en ce qu'**elle présente une sélectivité d'au moins 1000.

5. Membrane zéolithique supportée selon l'une des revendications 1 à 4 **caractérisée en ce que** la phase zéolithique contenue dans ladite couche composite est de type structural MFI.

6. Membrane zéolithique supportée selon l'une des revendications 1 à 5 **caractérisée en ce que** le support est choisi parmi les matériaux suivants : céramique à base d'alumine et/ou de zircone et/ou d'oxyde de titane, carbone, silice, zéolithes, argiles, verre poreux et métal poreux.

7. Procédé de préparation d'une membrane zéolithique supportée selon l'une des revendications 1 à 6 comprenant :
a) la formation d'un gel ou d'une solution comprenant une source de silice et de l'eau, additionné d'au moins un composé organique polaire ;
b) la mise en contact dudit gel ou de ladite solution avec un support poreux ;
c) la cristallisation de la zéolithe à partir dudit gel ou de ladite solution ; et
d) l'élimination d'agents résiduels ;

8. Procédé de préparation d'une membrane zéolithique supportée selon la revendication 7 **caractérisé en ce que** dans ladite étape a) la source de silice est une silice précipitée et le rapport molaire de l'eau à la silice est compris entre 45:1 et 65:1 et **en ce que** dans l'étape c) la durée de cristallisation est inférieure ou égale à 80 heures.

9. Procédé de préparation d'une membrane zéolithique supportée selon la revendication 7 **caractérisé en ce que** dans ladite étape a) la source de silice est une silice colloïdale et le rapport molaire de l'eau à la silice est compris entre 18:1 et 35:1 et **en ce que** dans l'étape c) la durée de cristallisation est inférieure ou égale à 45 heures.

10. Utilisation d'une membrane selon l'une des revendications 1 à 6 dans un procédé de séparation de gaz, de séparation de vapeurs ou de séparation de liquides.

11. Utilisation d'une membrane selon l'une des revendications 1 à 6 pour la séparation des paraffines linéaires et branchées.

12. Utilisation d'une membrane selon l'une des revendications 1 à 6 pour la séparation des paraffines branchées entre elles.

13. Utilisation d'une membrane selon l'une des revendications 1 à 6 pour la séparation des oléfines linéaires et branchées.

14. Utilisation d'une membrane selon l'une des revendications 1 à 6 pour la séparation des paraffines et des oléfines.

15. Utilisation d'une membrane selon l'une des revendications 1 à 6 pour la séparation des naphtènes et des paraffines.

16. Utilisation d'une membrane selon l'une des revendications 1 à 6 pour la séparation des paraffines et des aromatiques.

17. Utilisation d'une membrane selon l'une des revendications 1 à 6 pour la séparation de l'hydrogène et des hydrocarbures.

18. Utilisation d'une membrane selon l'une des revendications 1 à 6 pour la séparation des isomères du xylénes.

19. Utilisation d'une membrane selon l'une des revendications 1 à 6 pour la séparation du méthane et du CO₂.
